# EUROPEAN PATENT SPECIFICATION

(11) **EP 3 434 267 B1**
(45) Date of publication and mention of the grant of the patent: **26.07.2023**
(21) Application number: 17770126.5
(22) Date of filing: 16.03.2017
(51) Int. Cl.: A61K 9/70, A61K 31/485, A61P 25/04

(54) **PATCH PREPARATION HAVING MISUSE PREVENTION CHARACTERISTICS**
PFLASTERZUBEREITUNG MIT EIGENSCHAFTEN ZUR VERHINDERUNG VON MISSBRAUCH
PRÉPARATION DE PATCH PRÉSENTANT DES CARACTÉRISTIQUES DE PRÉVENTION D'UN MAUVAIS USAGE

(30) Priority: 24.03.2016 JP 2016060817
(43) Date of publication of application: 30.01.2019
(73) Proprietor: MEDRx Co., Ltd., Higashikagawa-shi Kagawa 769-2712 (JP)
(72) Inventor: HAMAMOTO, Hidetoshi, Higashikagawa-shi Kagawa 769-2712 (JP); MIWA, Yasushi, Higashikagawa-shi Kagawa 769-2712 (JP); YAMANAKA, Katsuhiro, Higashikagawa-shi Kagawa 769-2712 (JP); TATSUMI, Noboru, Higashikagawa-shi Kagawa 769-2712 (JP)
(74) Representative: Vossius & Partner Patentanwälte Rechtsanwälte mbB
(86) International application number: PCT/JP2017/010779
(87) International publication number: WO 2017/164084

(56) References cited:
- EP-A1- 2 865 376
- WO-A1-02/069942
- WO-A1-2015/037639
- WO-A1-2015/133329
- WO-A2-2007/070695
- WO-A2-2015/112563
- JP-A- H03 501 386
- JP-A- H07 504 882
- JP-A- 2006 525 316
- JP-A- 2009 519 958
- JP-A- 2010 229 045

## Description

### Technical Field

The present disclosure relates to a patch preparation having features that prevent misuse.

### Background Art

The misuse of pharmaceutical products including over-the-counter (OTC) drugs and prescription drugs has been the primary cause of poisoning in children worldwide for many years. In particular, patch preparations after use (i.e. patch preparations removed from the skin of a patient) can usually contain a certain amount of active ingredient. When these used preparations are applied to the skin of other people (in particular, children) unintentionally or intentionally, the preparations can cause any poisoning by the effect of the remaining active ingredient. The poisoning can occasionally lead to death. In order to prevent the careless misuse and/or the intentional abuse of pharmaceutical products, some techniques to properly dispose of patch preparations after use have been suggested (e.g. Patent Document 1).
Patent Document 2 describes a non-aqueous patch preparation composition containing a drug, an organic solvent, and a powder that is not soluble in a lipophilic base. Patent Document 3 discusses a composition for a non-aqueous patch preparation comprising a drug, an organic solvent, a lipophilic mass base, and a powder. Meanwhile, Patent Document 4 discusses formulations for dermal delivery of a drug, comprising: a) a drug; b) a solvent vehicle, comprising: i) a volatile solvent system including at least one volatile solvent, and ii) a nonvolatile solvent system that is flux-enabling for the drug; and c) a solidifying agent which contributes to solidification of a layer of the formulation applied on a skin surface upon at least partial evaporation of the volatile solvent system, and Patent Document 5 discloses a composition for application to a body surface or membrane to administer a drug by permeation through a body membrane comprising a drug and a permeation enhancing amount of a mixture of glycerol monooleate (GMO) and ethanol.

### Prior Art Documents

### Patent Documents

Patent Document 1: WO 2003/103673
Patent Document 2: WO 2015/037639 A1
Patent Document 3: EP 2 865 376 A1
Patent Document 4: WO 2007/070695 A2
Patent Document 5: JP H03 501386 A

### Summary of Invention

### (Problem to be Solved by the Invention)

An object of the present disclosure is to provide a patch preparation which can produce an effect for preventing the misuse after the removal of the patch preparation from the skin without any complicated operation for a patient.

### (Means for Solving the Problems)

The present inventors have studied a patch preparation having features that prevent misuse, and then have found that the patch preparation can be prepared by adding a solvent with a specific vapor pressure into the plaster therein.
The present inventors have completed the present invention on the basis of the above findings as defined in the appended claims.

The present disclosure is to provide the following (1) to (20).
(1) A patch preparation comprising a plaster disposed on a support, wherein the plaster comprises a solvent with a vapor pressure of 1 kPa or more at 20°C, and the amount of the solvent is about 2% to about 35% by weight of the plaster.
(2) The patch preparation of item (1), wherein the amount of solvent is about 5% to about 25% by weight of the plaster.
(3) The patch preparation of item (1) or (2), wherein the solvent is selected from an acyclic or cyclic aliphatic hydrocarbon, an aliphatic alcohol, an ester, a ketone, an ether, an aromatic hydrocarbon, water, or a combination thereof.
(4) The patch preparation of item (3), wherein the solvent is selected from n-hexane, n-heptane, cyclohexane, ethanol, isopropyl alcohol, isobutyl alcohol, 2-buthanol, isobutyl acetate, isopropyl acetate, ethyl acetate, acetone, methyl-ethyl-ketone, methyl-isobutyl-ketone, diethyl ether, tetrahydrofuran, 1,4-dioxane, benzene, xylene, water, or a combination thereof.
(5) The patch preparation of any one of items (1) to (4), wherein the plaster further comprises a medicament.
(6) The patch preparation of item (5), wherein the medicament is selected from a hypnosedative, a stimulant, a psychoneurotic agent, a regional anesthetic, a muscle relaxant suxametonium, an antiparkinsonian agent, an antimigraine agent, an anti-smoking drug, an anti-allergic agent, an anti-Alzheimer's agent, an opioid analgesic medication, or a combination thereof.
(7) The patch preparation of item (6), wherein the medicament comprises one or more opioid analgesic medications selected from morphine, codeine, fentanyl, oxycodone, hydromorphone, or a combination thereof.
(8) The patch preparation of any one of items (1) to (7), wherein the plaster further comprises a base material.
(9) The patch preparation of item (8), wherein the base material comprises an elastic polymer.
(10) The patch preparation of item (9), wherein the elastic polymer is selected from the group consisting of an acrylic polymer, a rubber polymer, and a silicone-based polymer.
(11) The patch preparation of any one of items (1) to (10), wherein the plaster further comprises one or more additives.
(12) The patch preparation of any one of items (1) to (11), wherein the support is a solvent-permeable support.
(13) The patch preparation of any one of items (1) to (11), wherein the support is a solvent-impermeable support.
(14) A patch preparation comprising a solvent-permeable support, a first plaster, a solvent-impermeable support, and a second plaster, wherein:
   the first plaster is disposed on the solvent-permeable support;
   the solvent-impermeable support is disposed on the first plaster;
   the second plaster is disposed on the solvent-impermeable support, and the second plaster comprises a medicament; and
   the surface area of the first plaster is greater than the surface area of the solvent-impermeable support, and the surface area of the solvent-impermeable support is equal to or greater than the surface area of the second plaster.
(15) The patch preparation of item (14), wherein the first plaster comprises no medicament.
(16) A method of preparing a patch preparation comprising the steps of:
   providing a support;
   providing a coating solution comprising a solvent and a polymer;
   coating the support with the coating solution to provide a plaster disposed on the support; and
   drying the plaster until the amount of the solvent reaches about 2% to about 35% by weight of the plaster.
(17) The method of item (16), wherein the coating solution further comprises a medicament.
(18) The method of item (16) or (17), wherein the solvent has a vapor pressure of 1 kPa or more at 20°C.
(19) The patch preparation of any one of items (1) to (13) for percutaneous delivery of a medicament, wherein the adhesibility of the patch preparation is eliminated when the plaster is exposed to air directly or through the solvent-permeable support to reduce the amount of the solvent in the plaster by more than 50%.
(20) The patch preparation of any one of items (1) to (13) for percutaneous delivery of a medicament, wherein the adhesibility of the patch preparation is eliminated when the plaster is exposed to air for at least about 10 minutes.

### BRIEF DESCRIPTION OF THE DRAWINGS

FIG. 1 shows a schematic cross-sectional view of an example of patch preparations with multi-layer structure.
FIG. 2 shows the percutaneous permeability of oxycodone in the patch preparation 6 evaluated using a Franz diffusion cell at each sampling point (1 hr, 2 hr, 4 hr, 6 hr, and 8 hr).
FIG. 3 shows the percutaneous permeability of fentanyl in the patch preparation 7 evaluated using a Franz diffusion cell at each sampling point (2 hr, 4 hr, 6 hr, 8 hr, and 24 hr).
FIG. 4 shows the percutaneous permeability of hydromorphone in the patch preparation 8 evaluated using a Franz diffusion cell at each sampling point (1 hr, 2 hr, 4 hr, 8 hr, 11 hr, 21 hr, and 24 hr).

### DETAILED DESCRIPTION OF THE PREFERRED EMBODIMENTS

The patch preparation of the present disclosure comprises a support and one or more plasters. The plaster comprises a solvent and a base material. In some embodiments, the plaster may comprise an active ingredient or a medicament, a solvent and a base material. In some embodiments, the plaster may further comprise one or more additives.

### Solvent

The plaster comprises a solvent with a vapor pressure of 1 kPa or more at 20°C. The amount of the solvent in the plaster is about 2 wt% to about 35 wt% of the plaster. Examples of the solvent include, but not covered by the claims, an acyclic or cyclic aliphatic hydrocarbon such as n-hexane, n-heptane, and cyclohexane; an aliphatic alcohol such as ethanol, isopropyl alcohol, isobutyl alcohol, and 2-buthanol; an ester such as isobutyl acetate, isopropyl acetate, and ethyl acetate; a ketone such as acetone, methyl-ethyl-ketone, and methyl-isobutyl-ketone; an ether such as diethyl ether, tetrahydrofuran and 1,4-dioxane; an aromatic hydrocarbon such as benzene and xylene; an inorganic solvent such as water. These solvents may be used alone or in suitable combination. In the present invention, the solvent is a combination of ethyl acetate and n-heptane.

The solvent with a vapor pressure of 1kPa or more at 20°C is rapidly evaporated from the plaster at room temperature (about 25°C) or skin surface temperature (about 32°C) to reduce or eliminate the adhesibility of the plaster. As a result, the accident due to the reapplication of the used patch preparation to the skin can be prevented. In some embodiments where the plaster comprises a medicament, the reduction in the amount of the solvent for dissolving the medicament can lead to a reduction or loss of the skin permeability of the medicament.

In some embodiments, the amount of the solvent with a vapor pressure of 1 kPa or more at 20°C is about 2% to about 35% by weight, but may be about 3% to about 20% by weight, or about 4% to about 12% by weight, relative to the total weight of the dried plaster. In some embodiments, the amount of the solvent may be about 5% to about 35% by weight, about 10% to about 35% by weight, about 12% to about 30% by weight, or about 15% to about 28% by weight, relative to the total weight of the dried plaster. In some embodiments where the plaster comprises no medicament and/or the base material is an acrylic polymer, the amount of the solvent is about 2% to about 35% by weight, but may be about 4% to about 30% by weight, about 5% to about 25% by weight, or about 5% to about 12 % by weight, relative to the total weight of the dried plaster. When the amount of the solvent is lower than the above range, the desired effect for reducing the adhesibility of the patch preparation may not be achieved. Also, when the amount of the solvent exceeds the above range, the patch preparation may not be formed appropriately.

In some embodiments, the amount of the solvent is substantially kept at a constant level until the release film of the patch preparation is removed after the packaging of the prepared patch preparation. In some embodiments, the amount of the solvent decreases by less than about 5%, less than about 3%, less than about 2%, or less than about 1%. As used herein, the amount of the solvent in the prepared patch preparation after packaging is measured after the coating and drying steps for providing the plaster. That is, it is not the amount of the solvent in the coating solution that is initially coated onto the support in the preparation of the plaster (charged amount).

Some attempts to keep the amount of the solvent in the patch preparation at a constant level can be made. For example, a patch preparation is wrapped in a wrapping material or is enclosed in a sealed packaging. Before the use of the patch preparation, the surface of the plaster (opposite to the support) is usually protected by the treated release film (release liner). In some embodiments, the release film may be impermeable to the solvent.

### Base Material

The base material may comprise a polymer. In some embodiments, the base material comprises an elastic polymer. Examples of the elastic polymer may include, but are not limited to, an acrylic polymer, a rubber polymer, and a silicone-based polymer. Preferably, the rubber polymer such as styrene-isoprene-styrene block copolymer, styrenebutadiene-styrene block copolymer, polyisoprene, polyisobutylene, and polybutadiene may be used. When a rubber polymer is used, the amount of the rubber polymer may be about 3% to about 40% by weight, about 4% to about 30% by weight, about 5% to about 20%, or about 5% to about 12% by weight, relative to the total weight of the plaster. When an acrylic polymer is used, the amount of the acrylic polymer may be about 15% to about 80% by weight, about 20% to 70% by weight, or about 30% to about 60%, relative to the total weight of the plaster.

### Medicament

The patch preparation of the present disclosure may comprise a medicament in the plaster. The medicament may include any active ingredient that is suitable for a patch preparation, and the type of medicament is not limiting. Examples of the medicament may include a hypnosedative, a stimulant, a psychoneurotic agent, a regional anesthetic, a muscle relaxant suxametonium, an antiparkinsonian agent, an antimigraine agent, an anti-smoking drug, an anti-allergic agent, an anti-Alzheimer's agent, and an opioid analgesic medication. Examples of the opioid analgesic medication may include morphine, codeine, fentanyl, oxycodone, and hydromorphone. These medicaments may be used in suitable combination. These medicaments are easily abused, and thus there is a high demand for preventing the abuse.

### Additive

In some embodiments, the patch preparation may further comprise one or more additives. For example, the plaster may further comprise an agent for preventing accidental ingestion. Examples of the agent may include a substance with strong bitter taste such as denatonium benzoate. When the amount of denatonium benzoate is about 0.001% to about 0.1% by weight or about 0.001 % to about 0.01% by weight relative to the total weight of the plaster, the patch preparation produces strong bitter taste in mouth to exert an effect for preventing the accidental ingestion for people (including children) and some animals.

### Tackifier

In some embodiments, the plaster may further comprise a tackifier. Examples of the tackifier may include rosin ester, hydrogenated rosin ester, maleate rosin, alicyclic saturated hydrocarbon resin, terpene resin, and polyolefin resin. The amount of the tackifier may be about 10% to about 40% by weight, about 20% to about 30% by weight, or about 22% to about 28% by weight, relative to the total weight of the plaster.

### Percutaneous Absorption Accelerator

In some embodiments, the plaster may further comprise one or more organic solvents with an effect for accelerating percutaneous absorption of a medicament. Examples of the organic solvent may include a fatty acid, a fatty alcohol, an ester, and an organic amine compound. Examples of the fatty acid may include capric acid, sorbic acid, levulinic acid, lauric acid, myristic acid, stearic acid, isostearic acid, and oleic acid. Examples of the fatty alcohol may include monovalent alcohol such as capryl alcohol, lauryl alcohol, myristyl alcohol, cetyl alcohol, stearyl alcohol, and oleyl alcohol; and polyalcohol such as propylene glycol, polyethylene glycol, and glycerin. Examples of the ester may include propylene carbonate, diethyl sebacate, isopropyl myristate, diisopropyl adipate, myristyl palmitate, stearyl stearate, and C₅₋₁₂ medium-chain triglyceride. Examples of the organic amine compound may include monoethanolamine, monoisopropanolamine, diethanolamine, diisopropanolamine, triethanolamine, and triisopropanolamine.

In some embodiments, the solvent in the plaster of the present disclosure is preferably a solvent with the effect for accelerating percutaneous permeation of a medicament and a vapor pressure of 1kPa or more at 20°C. For example, when oxycodone is used as an active ingredient in the patch preparation, ethyl acetate and n-heptane is used as the solvent. When the combination of ethyl acetate and n-heptane is used, the ratio between ethyl acetate and n-heptane may be, for example, 1:2 to 10:1. In some embodiments where the medicament is fentanyl, ethanol, isopropanol, or ethyl acetate may be used as the solvent.

### Other Additive

The plaster may also comprise a well-known additive such as a filler, a softener, an antioxidant agent, a pH adjuster, and a flavor.

### Support

The patch preparation comprises the plaster disposed on one side of the support. The support can be a support which is permeable to the solvent with a vapor pressure of 1kPa or more at 20°C (hereinafter, referred to as "solvent-permeable support"), or a support which is impermeable to the solvent with a vapor pressure of 1kPa or more at 20°C (hereinafter, referred to as "solvent-impermeable support").

Examples of the solvent-permeable support may include a woven or non-woven cloth and a porous sheet which comprise a material such as resin (e.g. polyester, polypropylene, polyurethane and vinyl chloride) and pulp. Examples of the solvent-impermeable support may include a film comprising a resin such as PET (polyethylene terephthalate), polyamide and vinyl chloride, and a film comprising a woven or non-woven cloth coated with a resin such as PET.

When the support is the solvent-permeable support, the evaporation of the solvent in the plaster can be prevented by any means such as wrapping the patch preparation with a suitable wrapping material until the patch preparation is applied to the skin. The amount of the solvent with a vapor pressure of 1 kPa or more at 20°C in the plaster is kept in a predetermined range until the release film is removed. The adhesibility of the plaster under this condition is sufficient, and thus the plater is attached to the skin and the adhesive state between the plater and the skin is kept. During the adhesion to the skin, the solvent is evaporated from the plaster through the solvent-permeable support. Even if the solvent is evaporated, the patch preparation can keep the adhesion to the skin. On the other hand, when the patch preparation in the state that the solvent has been evaporated from the plaster is removed from the skin, the patch preparation is not attached to the skin due to the loss or reduction of the adhesibility of the plaster. As used herein, the term "state that the solvent has been evaporated from the plaster" means that the amount of the solvent with a vapor pressure of 1 kPa or more at 20°C reaches less than about 50%, less than about 60%, less than about 70%, or less than about 75% of the solvent at the time of removing the release film.

In other words, when more than about 50%, about 60%, about 70%, or about 75%of the solvent has been evaporated from the plaster, the patch preparation is not attached to the skin. However, in some embodiments where the patch preparation has already been attached to the skin, the patch preparation can keep the adhesion to the skin even if the solvent is evaporated, for example, through the solvent-permeable support. In these embodiments, the adhesibility of the patch preparation is reduced or eliminated immediately after or after a while the patch preparation is removed.

In some embodiments, the adhesibility of the patch preparation is reduced or eliminated when the plaster is exposed to air for at least about 5 minutes, about 10 minutes, about 15 minutes, or about 30 minutes.

In some embodiments where the support is the solvent-impermeable support, the evaporation of the solvent in the plaster can be prevented by any means such as wrapping the patch preparation with a suitable wrapping material until the patch preparation is applied to the skin. The amount of the solvent in the plaster disposed on the solvent-impermeable support is kept at a constant level until the release film is removed. The plaster is disposed on the solvent-impermeable support on one side and is attached to the skin on the other side, and thus the amount of the solvent during the adhesion to the skin is not substantially changed. On the other hand, the plaster is warmed to near skin surface temperature (about 32°C) by body heat when the adhesion of the plaster to the skin is maintained, and thus the solvent in the plaster is rapidly evaporated after the plaster is removed from the skin. Thus, the adhesibility of the plaster after the removal is reduced or eliminated, and the plaster cannot be attached to the skin.

In some embodiments where the plaster comprises a medicament dissolved in a solvent, the medicament hardly penetrate the skin when the patch preparation is removed and the plaster with no solvent is attached to the skin.

In some embodiments, the solvent-permeable support and the solvent-impermeable support can be used in combination. The patch preparation can be a multi-layer structure comprising first and second plasters, and first and second supports. The first support is a solvent-permeable support, and the second support is a solvent-impermeable support. In some embodiments, the first plaster does not comprise any medicament, and second plaster comprises one or more medicaments. In some embodiments, the first support, the first plaster, the second support, and then the second plaster are sequentially laminated so that the second plaster is attached to the skin. The surface areas of the first plaster, second support and second plaster satisfy the following formula.
first plaster > second support ≥ second plaster

In some embodiments, the surface areas of the supports and plasters satisfy the following formula.
first support ≥ first plaster > second support ≥ second plaster

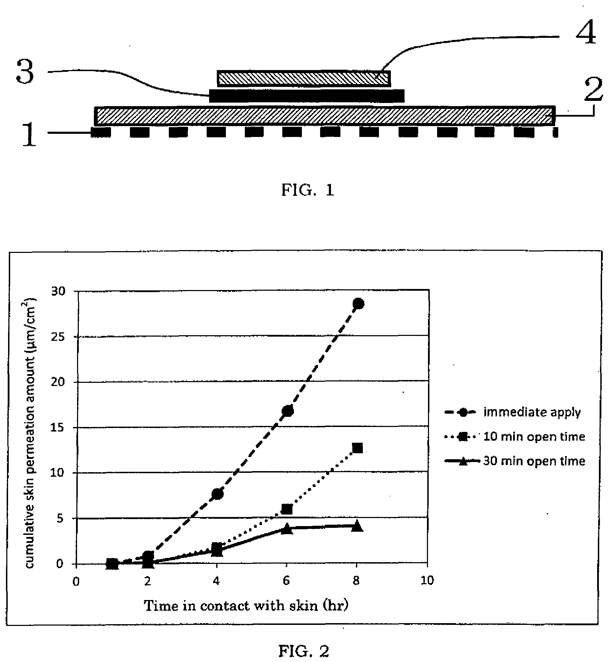

An Example of the patch preparations with multi-layer structure as described herein is further illustrated in FIG.1. FIG. 1 shows a schematic cross-sectional view of an example of the patch preparations with multi-layer structure of the present disclosure. In FIG.1, element 1 illustrates a solvent-permeable support, element 3 illustrates a solvent-impermeable support, and elements 2 and 4 illustrate a first plaster and a second plaster, respectively. As shown in FIG.1, the first plaster (2) is disposed on the solvent-permeable support (1), the solvent-impermeable support (3) is disposed on the first plaster (2), and the second plaster (4) is disposed on the solvent-impermeable support (3). The second plaster (4) comprises a medicament.

The first plaster plays a role in applying the patch preparation to the skin, and the second plaster plays a role in releasing a medicament comprised in the plaster after applying the patch preparation to the skin. Therefore, the surface area of the first plaster is preferably larger as compared to the surface area of the second support and the second plaster. Specifically, the surface area of the second support may be less than about 75%, less than about 60%, less than about 50%, or less than about 30% of that of the first support. In some embodiments, the adhesibility of the second plaster may be eliminated. In some embodiments, the adhesibility of the second plaster may be insufficient to keep the adhesion to the skin.

As described herein, the adhesibility of the first plaster is sufficient to keep the adhesion to the skin even if the patch preparation is applied to the skin. The first plaster is attached to the skin on one side, and is disposed on the solvent-permeable support on the other side. When the plaster is attached to the skin, the solvent with a vapor pressure of 1 kPa or more at 20°C in the plaster is evaporated through the solvent-permeable support. The solvent is rapidly evaporated within about 5 minutes, about 10 minutes, about 15 minutes, or about 30 minutes after applying to the skin. As a result, the adhesibility of the plaster is eliminated when the plaster is removed from the skin, and the plaster cannot be attached to the skin.

On the other hand, even if 30 minutes or more, 8 hours or more, 12 hours or more, and 24 hours or more have passed after applying to the skin and the solvent in the plaster is evaporated, the adhesion to the skin is kept as long as the plaster is not removed. The second plaster is disposed on the solvent-impermeable support on one side. Thus, when the patch preparation is applied to the skin, the solvent is hardly evaporated from the second plaster. The second plaster comprises the medicament dissolved in the solvent with a vapor pressure of 1 kPa or more at 20°C, and thus the medicament can penetrate the skin. On the other hand, when the second plaster is removed, the solvent therein is rapidly evaporated. As a result, even if the second plaster is attached to the skin, the percutaneous permeability of the medicament is remarkably decreased.

### Method of Preparing the Patch Preparation

The patch preparation is prepared by coating a support with a coating liquid or coating solution, disposing a plaster on the support, and drying the plaster. In some embodiments, the patch preparation has a adhesive force or strength in the surface of the dried plaster.

One or more polymers are dissolved in a solvent to prepare a solution comprising a base material. In some embodiments, the solution is used as the coating solution. In some embodiments, a solution of an active ingredient and the base material solution are combined to prepare the coating solution.

The active ingredient/medicament is dissolved in a solvent to prepare an active ingredient/medicament solution. The solvent may be the solvent described herein. In some embodiments, the solvent may comprise a solvent that can serve as a percutaneous absorption accelerator. In some embodiments, the medicament solution may comprise one or more additives.

The plaster is prepared by disposing or coating the coating solution onto a support. The plaster is then dried until the amount of solvent in the plaster reaches the selected range described herein. For example, the plaster is dried until the amount of the solvent reaches about 2% to about 30% by weight of the plaster.

The release film is applied to the surface of the exposed plaster when the amount of the solvent in the plaster reaches a desired amount. The patch preparation may be packaged.

### EXAMPLES

Hereinafter, the present disclosure will be described more specifically with reference to Examples. However, the present disclosure is not intended to be limited to them by any means.

### Patch Preparations 1-A and 1-B Containing Oxycodone

The patch preparations containing oxycodone with the composition shown in Table 1 below were prepared as follows. Potassium hydroxide, levulinic acid, and glycerin were mixed with heating, and then sodium hydrogen sulfite was added thereto and mixed to prepare a mixture thereof. To the prepared mixture were added oleic acid, denatonium benzoate, diisopropanolamine, propylene carbonate, and propyl gallate and mixed with heating, and then oxycodone hydrochloride was added thereto and mixed with heating to prepare an oxycodone solution. Separately from the preparation of the oxycodone solution, terpene resin, styrene-isoprene-styrene block copolymer, light anhydrous silicic acid, ethyl acetate, and heptane were mixed together to prepare a viscous elastomer solution. The oxycodone solution and the elastomer solution, which were separately prepared, were mixed to prepare a coating solution. The coating solution was coated on a PET liner treated with silicon, and dried until the amounts of ethyl acetate and heptane relative to the total weight of the plaster (adhesive layer) reach about 10% by weight. The PET liner was then laminated to the PET backing film (solvent-impermeable support) to prepare the patch preparations comprising a release liner. In addition, in the patch preparation 1-B, the release liner was removed to expose the plaster, and the exposed plaster was placed at room temperature for 1 hour to evaporate ethyl acetate and heptane from the plaster. After one hour, the adhesibility of the patch preparations was evaluated with one hand wearing a rubber glove. The adhesibility of the patch preparation 1-A was sufficient, whereas that of the patch preparation 1-B was eliminated.

### Patch Preparations 2-A and 2-B Containing Oxycodone

The patch preparations 2-A and 2-B were prepared following the same procedures as the patch preparations 1-A and 1-B, except that the amount of the ingredients with the composition shown in Table 1 below for the patch preparations 2-A and 2-B were used, and the amounts of ethyl acetate and heptane after drying were adjusted to 5%, respectively. In addition, in the patch preparation 1-B, the release liner was removed to expose the plaster, and the exposed plaster was placed at room temperature for 1 hour to evaporate ethyl acetate and heptane from the plaster. After one hour, the adhesibility of the patch preparations was evaluated with one hand wearing a rubber glove. The adhesibility of the patch preparation 1-A was sufficient, whereas that of the patch preparation 1-B was eliminated.

### In vitro Evaluation of Percutaneous Permeability

The percutaneous permeability of oxycodone in the patch preparations 1-A to 2-B was evaluated using a Franz diffusion cell. The evaluation was performed in the skin of a pig. The cumulative skin permeation amount at each sampling point (2 hr, 4 hr, 6 hr, and 8 hr) is shown in Table 1 below. As shown in Table 1, the patch preparations 1-B and 2-B were much less the cumulative skin permeation amount at each sampling point as compared to the patch preparations 1-A and 2-A.

**[Table 1]**

| | | 1-A | 1-B | 2-A | 2-B |
|---|---|---|---|---|---|
| Oxycodone hydrochloride ·3H₂O | | 18.64 | 18.64 | 19.62 | 19.62 |
| ethyl acetate | | 372.88 | 372.88 | 392.39 | 392.39 |
| heptane | | 124.29 | 124.29 | 130.80 | 130.80 |
| glycerin | | 15.54 | 15.54 | 16.35 | 16.35 |
| oleic acid | | 24.86 | 24.86 | 26.16 | 26.16 |
| levulinic acid | | 6.21 | 6.21 | 6.54 | 6.54 |
| diisopropanolamine | | 4.35 | 4.35 | 4.58 | 4.58 |
| propylene carbonate | | 31.07 | 31.07 | 32.70 | 32.70 |
| terpene resin | | 273.44 | 273.44 | 261.59 | 261.59 |
| SIS copolymer | | 87.01 | 87.01 | 65.40 | 65.40 |
| potassium hydroxide | | 3.42 | 3.42 | 3.60 | 3.60 |
| light anhydrous silicic acid | | 37.29 | 37.29 | 39.24 | 39.24 |
| denatonium benzoate | | 0.062 | 0.062 | 0.065 | 0.065 |
| sodium hydrogen sulfite | | 0.62 | 0.62 | 0.65 | 0.65 |
| propyl gallate | | 0.31 | 0.31 | 0.33 | 0.33 |
| total | | 1000 | 1000 | 1000 | 1000 |
| ethyl acetate content in the plaster after drying | | 10% | 10% | 5% | 5% |
| heptane content in the plaster after drying | | 10% | 10% | 5% | 5% |
| adhesibility | | ○ | × | ○ | × |
| cumulative skin permeation amount (µg/cm²) | 2 hr | 6.90 | 0.00 | 2.10 | 0.80 |
| | 4 hr | 16.50 | 1.10 | 12.50 | 5.20 |
| | 6 hr | 29.00 | 4.30 | 27.60 | 12.20 |
| | 8 hr | 42.90 | 9.90 | 44.90 | 21.30 |

| | | | | | |
|---|---|---|---|---|---|
| O : The patch preparation had the adhesibility in the plaster. × : The patch preparation had no adhesibility in the plaster. SIS copolymer : styrene-isoprene-styrene copolymer | | | | | |

### Patch Preparations 3 and 4 Containing Oxycodone

The patch preparations 3 and 4 were prepared following the same procedure as the patch preparation 1-A, except that the ingredients with the composition shown in Table 2 below were used, and a solvent-permeable woven cloth was used instead of the PET liner. Each of the patch preparations 3 and 4 is applied to the skin at room temperature for 30 minutes, and then is removed from the skin. The patch preparations 3 and 4 are then immediately applied to the skin again. However, the patch preparations 3 and 4 are not reattached to the skin. This demonstrates that the patch preparations have the desired function for safety.

### Patch Preparation 5 Containing Oxycodone

The patch preparation 5 was prepared following the same procedure as the patch preparation 1-A, except that the ingredients with the composition shown in Table 2 below were used. The patch preparation 5 is applied to the skin at room temperature for 30 minutes. The amount of the solvent in the plaster of the patch preparation 5 is kept at a constant level while being applied to the skin. The patch preparation 5 is removed from the skin, and then the patch preparation is applied to skin again after 30 minutes. However, the patch preparation 5 cannot be reattached to skin since the solvent is rapidly evaporated from the plaster immediately after the patch preparation 5 is removed from the skin.

**[Table 2]**

| | 3 | 4 | 5 |
|---|---|---|---|
| | U830 | U829 | R814P |
| Oxycodone hydrochloride·3H₂O | 21.6 | 21.2 | 18.4 |
| ethyl acetate | 375.3 | 367.3 | 371.6 |
| heptane | 125.1 | 122.4 | 185.8 |
| glycerin | 0.0 | 15.3 | 13.3 |
| oleic acid | 25.0 | 24.5 | 21.2 |
| levulinic acid | | 6.1 | 5.3 |
| diisoproopanolamine | 4.4 | 4.3 | 3.7 |
| propylene carbonate | 31.3 | 30.6 | 26.5 |
| medium-chain triglyceride | | 30.6 | |
| oleyl alcohol | 31.3 | | |
| terpene resin | 250.2 | 244.8 | 254.8 |
| SIS copolymer | 93.8 | 91.8 | 63.7 |
| potassium hydroxide | 3.4 | 3.4 | 2.9 |
| light ahydrous silicic acid | 37.5 | 36.7 | 31.9 |
| denatonium benzoate | 0.0626 | 0.0612 | 0.0531 |
| sodium hydrogen sulfite | | | 0.53 |
| propyl gallate | 0.31 | 0.31 | 0.27 |
| sodium pyrosulfite | 0.63 | 0.61 | |
| total | 1000 | 1000 | 1000 |
| ethyl acetate content after drying | 8% | 8% | 3% |
| heptane content after drying | 3% | 3% | 4% |

### Patch Preparation 6 Containing Oxycodone

The patch preparation 6 was prepared following the same procedure as the patch preparation 1-A, except that the ingredients with the composition shown in Table 3 below were used. The percutaneous permeability of oxycodone in the patch preparation 6 was evaluated using a Franz diffusion cell. The evaluation was performed in the skin of a pig. The cumulative skin permeation amount at each sampling point (1 hr, 2 hr, 4 hr, 6 hr, and 8 hr) is shown in Table 4 and FIG. 2.

Separately from the above evaluation, the release liner of the patch preparation 6 was removed, and one side of the plaster was exposed to air at room temperature for 10 minutes or 30 minutes to evaporate ethyl acetate and heptane from the plaster, and then the percutaneous permeability of oxycodone in the patch preparation 6 was evaluated in the skin of a pig using a Franz diffusion cell. The cumulative skin permeation amount at each sampling point (1 hr, 2 hr, 4 hr, 6 hr, and 8 hr) is shown in Table 4 and FIG. 2. As shown in FIG. 2, the patch preparation 6 exposed to air for 10 minutes or 30 minutes was much less cumulative skin permeation amount as compared to the patch preparation 6 applied to the skin immediately.

**[Table 3]**

| Patch Preparation 6 | Amount in the plaster |
|---|---|
| oxycodone hydrochloride•3H₂O | 3.016 |
| ethyl acetate | 16.0 |
| heptane | 7.0 |
| glycerin | 2.178 |
| oleic acid | 3.485 |
| levulinic acid | 0.871 |
| diisopropanolamine | 0.610 |
| propylene carbonate | 4.357 |
| polyisobutylene | 1.743 |
| SIS copolymer | 47.923 |
| terpene resin | 9.585 |
| potassium hydrate | 0.497 |
| light anhydrous silicic acid | 2.614 |
| denatonium benzoate | 0.009 |
| sodium hydrogen sulfite | 0.087 |
| propyl gallate | 0.044 |
| Total | 100.02 |

**[Table 4]**

| Patch Preparation 6 | | 1 hr | 2 hr | 4 hr | 6 hr | 8 hr |
|---|---|---|---|---|---|---|
| cumulative skin permeation amount (µg/cm²) | immediate apply | 0 | 0.8 | 7.6 | 16.7 | 28.5 |
| | 10 min open time | 0 | 0.0 | 1.7 | 5.9 | 12.6 |
| | 30 min open time | 0 | 0.1 | 1.4 | 3.8 | 4.1 |

### Patch Preparation 7 Containing Fentanyl

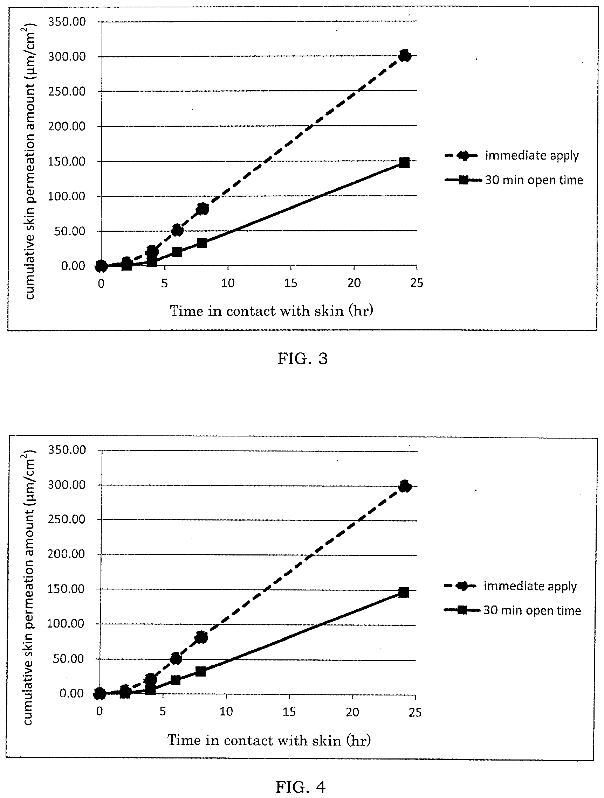

The patch preparation 7 was prepared following the same procedure as the patch preparation 1-A, except that fentanyl was used as an active ingredient instead of oxycodone hydrochloride, and the ingredients with the composition shown in Table 5 below were used. In Table 5, the term "charged amount" refers to the amount of each ingredient used to prepare the coating solution, and the term "amount after drying" refers to the amount of each ingredient after the coating solution is coated on a PET liner and dried. The percutaneous permeability of fentanyl in the patch preparation 7 was evaluated using a Franz diffusion cell. The evaluation was performed in the skin of a pig. The cumulative skin permeation amount at each sampling point (2 hr, 4 hr, 6 hr, 8 hr, and 24 hr) is shown in Table 6 and FIG. 3.

Separately from the above evaluation, the release liner of the patch preparation 7 was removed, and one side of the plaster was exposed to air at room temperature for 30 minutes to evaporate ethyl acetate and heptane from the plaster, and then the percutaneous permeability of fentanyl in the patch preparation 7 was evaluated in the skin of a pig using a Franz diffusion cell. The cumulative skin permeation amount at each sampling point (2 hr, 4 hr, 6 hr, 8 hr, and 24 hr) is shown in Table 5 and FIG. 3. As shown in FIG. 3, the patch preparation 7 exposed to air for 30 minutes was much less cumulative skin permeation amount as compared to the patch preparation 7 applied to the skin immediately.

**[Table 5]**

| Patch Preparation 7 | Charged amount (wt%) | Amount after drying (wt%) |
|---|---|---|
| fentanyl | 8.00 | 8.09 |
| ethyl acetate | 91.620 | 14.09 |
| heptane | 36.00 | 9.05 |
| polyisobutylene | 1.98 | 2.000 |
| SIS copolymer | 55.00 | 55.600 |
| terpene resin | 11.00 | 11.120 |
| propyl gallate | 0.05 | 0.050 |
| Total | 203.65 | 100.00 |

**[Table 6]**

| Patch Preparation 7 | | 0 hr | 2 hr | 4 hr | 6 hr | 8 hr | 24 hr |
|---|---|---|---|---|---|---|---|
| cumulative skin permeation amount (µg/cm²) | immediate apply | 0.00 | 4.20 | 20.76 | 51.26 | 81.50 | 300.39 |
| | 30 min open time | 0.00 | 0.71 | 5.50 | 19.54 | 32.66 | 147.27 |

### Patch Preparation 8 Containing Hydromorphone

The patch preparation 8 was prepared following the same procedure as the patch preparation 1-A, except that hydromorphone hydrochloride was used as an active ingredient instead of oxycodone hydrochloride, and the ingredients with the composition shown in Table 7 below were used. In Table 8, the term "charged amount" refers to the amount of each ingredient used to prepare the coating solution, the term "amount after drying" refers to the amount of each ingredient after the coating solution is coated on a PET liner and dried, and the term "amount after 30 minutes" refers to the amount of each ingredient after the PET liner is exposed to air for 30 minutes. The percutaneous permeability of hydromorphone of the patch preparation 8 was evaluated using a Franz diffusion cell. The evaluation was performed in the skin of a pig. The cumulative skin permeation amount at each sampling point (1 hr, 2 hr, 4 hr, 8 hr, 11 hr, 21 hr, and 24 hr) is shown in Table 8 and FIG. 4.

Separately from the above evaluation, the release liner of the patch preparation 8 was removed, and one side of the plaster was exposed to air at room temperature for 30 minutes to evaporate ethyl acetate and heptane from the plaster, and then the percutaneous permeability of fentanyl in the patch preparation 8 was evaluated in the skin of a pig using a Franz diffusion cell.. The cumulative skin permeation amount at each sampling point (2 hr, 4 hr, 6 hr, 8 hr, and 24 hr) is shown in Table 8 and FIG. 4. As shown in Fig. 4, the patch preparation 8 exposed to air for 30 minutes was much less cumulative skin permeation amount as compared to the patch preparation 7 applied to the skin immediately.

**[Table 7]**

| Patch Preparation 8 | Charged amount (wt%) | Amount after drying (wt%) | Amount after 30 mins (wt%) |
|---|---|---|---|
| hydromorphone hydrochloride | 3.00 | 2.83 | 2.59 |
| ethyl acetate | 75.00 | 17.89 | 0.52 |
| heptane | 5.00 | 0.98 | 0.27 |
| oleic acid | 2.00 | 1.89 | 1.73 |
| diisopropanolamine | 1.00 | 0.94 | 0.86 |
| glyceryl monostearate | 0.50 | 0.47 | 0.43 |
| HCO-40 | 1.50 | 1.42 | 1.29 |
| MCT | 2.00 | 1.89 | 1.73 |
| isopropyl myristate | 1.00 | 0.94 | 0.86 |
| diethyl sebacate | 1.00 | 0.94 | 0.86 |
| glycerine | 4.00 | 3.78 | 3.45 |
| 1,3-butanediol | 4.00 | 3.78 | 3.45 |
| oleyl alcohol | 6.00 | 5.66 | 5.18 |
| terpene resin | 10.00 | 9.44 | 8.63 |
| SIS copolymer | 44.00 | 41.53 | 37.95 |
| polyisobutylene | 2.00 | 1.89 | 1.73 |
| light anhydrous silicic acid | 3.00 | 2.83 | 2.59 |
| denatonium benzoate | 0.01 | 0.01 | 0.14 |
| sodium hydrogen sulfite | 0.20 | 0.19 | 0.17 |
| propyl gallate | 0.50 | 0.47 | 0.43 |
| BHT | 0.25 | 0.24 | 0.21 |
| total | 165.96 | 100.01 | 75.07 |

**[Table 8]**

| Patch Preparation 8 | | 0hr | 1hr | 2hr | 4hr | 8hr | 11hr | 21hr | 24hr |
|---|---|---|---|---|---|---|---|---|---|
| cumulative skin | immediate apply | 0.00 | 11.24 | 14.10 | 21.01 | 42.45 | 62.20 | 132.16 | 152.84 |
| permeation amount (µg/cm²) | 30 min open time | 0.00 | 10.58 | 11.31 | 13.14 | 18.99 | 26.59 | 64.19 | 76.56 |

### Patch Preparation 9 Containing No Active Ingredient (Reference Example)

An appropriate amount of ethyl acetate was added to a mixture of styrene-isopene-styrene block copolymer (6 parts by weight) and terpene resin (1 part by weight) was added to prepare a coating liquid with a suitable viscosity for coating. The coating liquid was coated onto a PET liner treated with silicon, and dried until the amount of ethyl acetate in the plaster achieved 10% by weight. The plaster was laminated onto a woven cloth backing (solvent-permeable support) to prepare the patch preparation 9. The prepared preparation was applied to the skin of human, and was removed from the skin after 30 minutes. The peeling stress was not small, but the plaster after the removal could not be reattached to the skin due to no adhesibility of the plaster.

### Patch Preparations 10 Containing No Active Ingredient

Ethyl acetate and heptane were added to a 1:1 (by weight) mixture of acrylic resin (DURO TAK) and terpene resin to prepare a coating liquid. The coating liquid was coated onto a PET liner treated with silicon, and dried until the amount of each ingredient achieved the ratio shown in Table 9 below. The plaster was laminated onto a woven cloth backing (solvent permeable support) to prepare the patch preparation 10-1. The patch preparation 10-2 was prepared following the same procedure as the patch preparation 10-1, except that denatonium benzoate was further added. The prepared patch preparations 10-1 and 10-2 were applied to the skin of human, and were removed from the skin after 30 minutes. The removed force was not small, but the plaster after the removal could not be reattached to the skin due to no adhesibility of the plaster.

**[Table 9]**

| | 10-1 | 10-2 |
|---|---|---|
| acrylic resin | 47 | 47 |
| terpen resin | 47 | 47 |
| ethyl acetate | 3 | 3 |
| heptane | 3 | 2.99 |
| denatonium benzoate | | 0.01 |
| total | 100 | 100 |

## Claims

1. A patch preparation comprising a plaster disposed on a support, wherein the plaster comprises a solvent with a vapor pressure of 1 kPa or more at 20°C, and the amount of the solvent is 2% to 35% by weight of the plaster, wherein the solvent is a combination of ethyl acetate and n-heptane.

2. The patch preparation of claim 1, wherein the amount of solvent is 5% to 25% by weight of the plaster.

3. The patch preparation of claim 1 or 2, wherein the plaster further comprises a medicament.

4. The patch preparation of claim 3, wherein the medicament is selected from a hypnosedative, a stimulant, a psychoneurotic agent, a regional anesthetic, a muscle relaxant suxamethonium, an antiparkinsonian agent, an antimigraine agent, an anti-smoking drug, an anti-allergic agent, an anti-Alzheimer's agent, an opioid analgesic medication, or a combination thereof.

5. The patch preparation of any one of claims 1 to 4, wherein the support is a solvent-permeable support.

6. The patch preparation of any one of claims 1 to 4, wherein the support is a solvent-impermeable support.

7. The patch preparation of claim 1 comprising a solvent-permeable support, a first plaster, a solvent-impermeable support, and a second plaster, wherein:
the first plaster is disposed on the solvent-permeable support;
the solvent-impermeable support is disposed on the first plaster;
the second plaster is disposed on the solvent-impermeable support, and the second plaster comprises a medicament; and
the surface area of the first plaster is greater than the surface area of the solvent-impermeable support, and the surface area of the solvent-impermeable support is equal to or greater than the surface area of the second plaster.

8. The patch preparation of claim 7, wherein the first plaster comprises no medicament.

9. A method of preparing a patch preparation comprising the steps of:
providing a support;
providing a coating solution comprising a solvent and a polymer, wherein the solvent is a combination of ethyl acetate and n-heptane;
coating the support with the coating solution to providing a plaster; and
drying the plaster until the amount of the solvent reaches 2% to 35% by weight of the plaster.

10. The method of claim 9, wherein the coating solution further comprises a medicament.

## Patentansprüche

1. Eine Pflasterzubereitung, umfassend ein auf einem Träger angeordnetes Pflaster, wobei das Pflaster ein Lösungsmittel mit einem Dampfdruck von 1 kPa oder mehr bei 20°C umfasst und die Menge des Lösungsmittels 2 bis 35 Gew.-% des Pflasters beträgt, wobei das Lösungsmittel eine Kombination von Ethylacetat und n-Heptan ist.

2. Die Pflasterzubereitung nach Anspruch 1, wobei die Menge an Lösungsmittel 5 bis 25 Gew.-% des Pflasters beträgt.

3. Die Pflasterzubereitung nach Anspruch 1 oder 2, wobei das Pflaster weiter ein Medikament umfasst.

4. Die Pflasterzubereitung nach Anspruch 3, wobei das Medikament ausgewählt ist aus einem Hypnosedativum, einem Stimulans, einem Psychoneurotikum, einem Regionalanästhetikum, einem muskelrelaxierenden Suxamethonium, einem Antiparkinsonmittel, einem Antimigränemittel, einem Anti-Raucher-Medikament, einem Antiallergikum, einem Mittel gegen Alzheimer, einer Opioidanalgetikum-Medikation oder einer Kombination davon.

5. Die Pflasterzubereitung nach einem der Ansprüche 1 bis 4, wobei der Träger ein lösungsmitteldurchlässiger Träger ist.

6. Die Pflasterzubereitung nach einem der Ansprüche 1 bis 4, wobei der Träger ein lösungsmittelundurchlässiger Träger ist.

7. Die Pflasterzubereitung nach Anspruch 1, umfassend einen lösungsmitteldurchlässigen Träger, ein erstes Pflaster, einen lösungsmittelundurchlässigen Träger und ein zweites Pflaster, wobei:
das erste Pflaster auf dem lösungsmitteldurchlässigen Träger angeordnet ist;
der lösungsmittelundurchlässige Träger auf dem ersten Pflaster angeordnet ist;
das zweite Pflaster auf dem lösungsmittelundurchlässigen Träger angeordnet ist und
das zweite Pflaster ein Medikament umfasst; und
die Oberfläche des ersten Pflasters größer ist als die Oberfläche des lösungsmittelundurchlässigen Trägers und die Oberfläche des lösungsmittelundurchlässigen Trägers gleich oder größer ist als die Oberfläche des zweiten Pflasters.

8. Die Pflasterzubereitung nach Anspruch 7, wobei das erste Pflaster kein Medikament umfasst.

9. Ein Verfahren zur Herstellung einer Pflasterzubereitung, umfassend die Schritte:
Bereitstellen eines Trägers;
Bereitstellen einer Beschichtungslösung, umfassend ein Lösungsmittel und ein Polymer, wobei das Lösungsmittel eine Kombination von Ethylacetat und n-Heptan ist;
Beschichten des Trägers mit der Beschichtungslösung, um ein Pflaster bereitzustellen; und
Trocknen des Pflasters, bis die Menge des Lösungsmittels 2 bis 35 Gew.-% des Pflasters erreicht.

10. Das Verfahren nach Anspruch 9, wobei die Beschichtungslösung weiter ein Medikament umfasst.

## Revendications

1. Préparation de patch comprenant un emplâtre disposé sur un support, dans laquelle l'emplâtre comprend un solvant avec une pression de vapeur de 1 kPa ou supérieure à 20°C, et la quantité du solvant est de 2 % à 35 % en masse de l'emplâtre, dans laquelle le solvant est une combinaison d'acétate d'éthyle et de n-heptane.

2. Préparation de patch selon la revendication 1, dans laquelle la quantité de solvant est de 5 % à 25 % en masse de l'emplâtre.

3. Préparation de patch selon la revendication 1 ou 2, dans laquelle l'emplâtre comprend de plus un médicament.

4. Préparation de patch selon la revendication 3, dans laquelle le médicament est choisi parmi un hypnosédatif, un stimulant, un agent psychoneurotique, un anesthésique local, un suxaméthonium de relaxation musculaire, un agent antiparkinsonien, un agent anti-migraine, un médicament anti-tabac, un agent anti-allergique, un agent anti-Alzheimer, un médicament analgésique d'opioïde, ou une combinaison de ceux-ci.

5. Préparation de patch selon l'une quelconque des revendications 1 à 4, dans laquelle le support est un support perméable au solvant.

6. Préparation de patch selon l'une quelconque des revendications 1 à 4, dans laquelle le support est un support imperméable au solvant.

7. Préparation de patch selon la revendication 1 comprenant un support perméable au solvant, un premier emplâtre, un support imperméable au solvant, et un second emplâtre, dans laquelle :
le premier emplâtre est disposé sur le support perméable au solvant ;
le support imperméable au solvant est disposé sur le premier emplâtre ;
le second emplâtre est disposé sur le support imperméable au solvant, et le second emplâtre comprend un médicament ; et la surface spécifique du premier emplâtre est supérieure à la surface spécifique du support imperméable au solvant, et la surface spécifique du support imperméable au solvant est supérieure ou égale à la surface spécifique du second emplâtre.

8. Préparation de patch selon la revendication 7, dans laquelle le premier emplâtre ne comprend pas de médicament.

9. Procédé de préparation d'une préparation de patch comprenant les étapes de :
fourniture d'un support ;
fourniture d'une solution de revêtement comprenant un solvant et un polymère, dans lequel le solvant est une combinaison d'acétate d'éthyle et de n-heptane ;
revêtement du support avec la solution de revêtement pour fournir un emplâtre ; et
séchage de l'emplâtre jusqu'à ce que la quantité du solvant atteigne de 2 % à 35 % en masse du emplâtre.

10. Procédé selon la revendication 9, dans lequel la solution de revêtement comprend de plus un médicament.
